# EUROPEAN PATENT SPECIFICATION

(11) **EP 4 135 581 B1**
(45) Date of publication and mention of the grant of the patent: **29.01.2025**
(21) Application number: 21788711.6
(22) Date of filing: 19.04.2021
(51) Int. Cl.: A61B 6/03, A61B 6/00

(54) **AN IMAGING SYSTEM ISOLATION ENCLOSURE FOR PATHOGEN CONTAINMENT DURING MEDICAL IMAGING PROCEDURES**
BILDGEBUNGSSYSTEMISOLATIONSGEHÄUSE ZUR KRANKHEITSERREGEREINDÄMMUNG WÄHREND MEDIZINISCHER BILDGEBUNGSVERFAHREN
ENCEINTE D'ISOLATION DE SYSTÈME D'IMAGERIE POUR ISOLATION DE PATHOGÈNES PENDANT DES PROCÉDURES D'IMAGERIE MÉDICALE

(30) Priority: 17.04.2020 US 202063011473 P
(43) Date of publication of application: 22.02.2023
(73) Proprietor: General Electric Company, Schenectady, NY 12345 (US)
(72) Inventor: FITZGERALD, Paul Francis, Schenectady, New York 12308 (US); WIEDMANN, Uwe, New York 12065 (US); STALTER, Ross Christopher, Hartland, Wisconsin 53029 (US); ARAUJO, Stephen Lorenco, Niskayuna, New York 12309 (US); RISHEL, Michael James, New York 12118 (US); SMITH, Chad Allan, Franklin, Wisconsin 53132 (US)
(74) Representative: AWA Sweden AB
(86) International application number: PCT/US2021/028015
(87) International publication number: WO 2021/212120

(56) References cited:
- EP-B1- 1 262 146
- DE-A1- 102012 217 445
- US-A- 2 915 074
- US-A- 3 710 791
- US-A- 4 676 258
- US-A1- 2004 202 287
- US-A1- 2004 228 450
- US-A1- 2014 378 817
- US-A1- 2020 054 299

## Description

### BACKGROUND

The present disclosure relates to an imaging system isolation enclosure, and more particularly to an imaging system isolation enclosure for use during medical imaging procedures.

In terms of advancements in technology, various non-invasive diagnostic scanning and imaging techniques are now utilized to aid with diagnosis and patient care. Exposure and spread of pathogens is of concern when such equipment is utilized in conjunction with patients having one or more infectious diseases. Contamination of medical imaging equipment and collateral equipment in the imaging suite, and general exposure to personnel within the imaging suite, with these pathogens presents a risk to health providers including imaging operators, patient caretakers, and field engineers when servicing the imaging components. In addition, repeat usage of contaminated medical imaging equipment within the imaging suite may present a risk to subsequent users and operators. Not only is the contamination of medical imaging system surfaces of concern, but in some instances, pathogens could be drawn into the imaging equipment's air intake, circulated within the equipment and ultimately circulated around the room by cooling-air exhaust fans. Similarly, exposure to a non-infectious patient by contaminated imaging equipment and/or a contaminated imaging suite is of concern.

It has been proposed that imaging systems could be sterilized using pathogen specific disinfectants, such as vaporized hydrogen peroxide (H₂O₂). Additionally, the imaging systems may be disinfected by using ultraviolet (UV) light cleaning systems to inactivate pathogens on the surfaces of the imaging systems. The need to clean imaging components, imaging systems and imaging suites to limit hospital acquired infections presents a burden and dramatically impacts patient throughput. In addition, cleaning medical imaging suites and the imaging systems contained therein with such vaporized solutions may result in the vaporized solutions being drawn into the imaging system by the system's cooling air exchange or, even if the cooling fans are turned off, the vaporized solution can make its way into the imaging system passively, as these vaporized solutions are designed to work. Consequently, the internal components of the equipment may become exposed to the vaporized solution, which can then condense on the internal components and may cause corrosion or other degradation of internal components of the imaging system.

Document DE 10 2012 217445 A1 discloses an imaging system isolation enclosure comprising: a pathogen impermeable enclosure for use with one or more of radiation imaging systems and magnetic resonance imaging systems, the pathogen impermeable enclosure configured to provide a barrier between the imaging system and at least one of a patient user and an imaging room.

It would therefore be desirable to provide a robust enclosure that isolates the medical imaging equipment from the patient, the healthcare professionals, and the imaging room or suite that addresses the above issues.

### SUMMARY

The invention is defined in independent claim 1, particular embodiments are defined in the independent claims.

### BRIEF DESCRIPTION OF THE DRAWINGS

The features, aspects, and advantages of the present disclosure will become better understood when the following detailed description is read with reference to the accompanying drawings, wherein:
FIG. 1 is a schematic perspective view of an embodiment of an imaging system for use with an imaging system isolation enclosure;
FIG. 2 is a schematic perspective view of an embodiment of an imaging system for use with an imaging system isolation enclosure;
FIG. 3 is a schematic perspective view of an embodiment of an imaging system isolation enclosure positioned relative to an imaging system; and
FIG. 4 is a schematic perspective view of an embodiment of an imaging system isolation enclosure positioned relative to an imaging system.

### DETAILED DESCRIPTION

The present disclosure includes an imaging system isolation enclosure for use with an imaging system to cover and contain therein at least a portion of the imaging system. The imaging system isolation enclosure is generally comprised as pathogen impermeable and compatible with one or more imaging systems and an air filtration system configured to supply filtered cooling air flow to an interior of the imaging system isolation enclosure and the imaging system. The imaging system isolation enclosure provides isolation of the medical imaging equipment, and in particular the imaging system, from pathogens that may be present in patients utilizing the imaging systems, as well as pathogens present in the surrounding imaging room or suite.

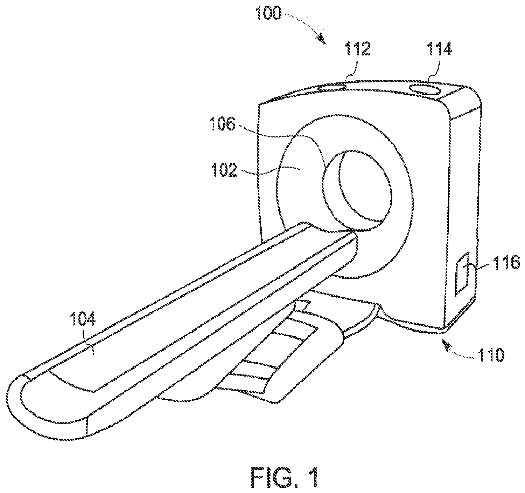

FIG. 1 illustrates an exemplary computed tomography (CT) imaging system 100 configured for CT imaging or scanning. Particularly, the CT imaging system 100 is configured to image a patient 110. In an exemplary embodiment, the CT imaging system 100 includes a gantry enclosure assembly 102 enclosing or housing a plurality of gantry components, which in turn, may further include at least one X-ray source (not shown) configured to emit a beam of X-ray radiation for use in imaging the patient (not shown) laying on a table 104 of the CT imaging system. The gantry enclosure assembly 102 includes a bore or opening 106 extending through the center of the gantry enclosure assembly 102. The X-ray source is configured to project an X-ray radiation beam towards an X-ray detector array (not shown) positioned directly opposite from the X-ray source in the gantry enclosure assembly 102.

In some CT imaging system configurations, an X-ray source emits a cone-shaped X-ray radiation beam which is collimated to lie within an x-y-z plane of a Cartesian coordinate system and is generally referred to as an "imaging plane." The X-ray radiation beam passes through the patient being imaged. The X-ray radiation beam, after being attenuated by the patient, is received by the X-ray detector array. The intensity of the attenuated X-ray radiation beam received at the X-ray detector array is dependent upon the attenuation of the X-ray radiation beam by the patient.

In some CT imaging systems, the X-ray source and the X-ray detector array are rotated within the gantry and around the patient being imaged creating an imaging plane, such that an angle at which the X-ray radiation beam intersects the patient constantly changes. A plurality of X-ray radiation attenuation measurements, e.g., projection data, from the X-ray detector array at any one gantry angle is referred to as a "view." A "scan" of the patient includes a set of views made at different gantry angles, or view angles, during one revolution of the X-ray source and X-ray detector array. As used herein the term "view" is not limited to the use as described above with respect to projection data from one gantry angle, but the term "view" is used to mean one data acquisition whenever there are multiple data acquisitions from different angles, whether from a CT imaging system, and/or any other imaging system, including imaging systems yet to be developed as well as combinations thereof.

The present disclosure includes a imaging system isolation enclosure for use with a medical imaging system to cover and contain therein at least a portion of an imaging system. The imaging system isolation enclosure is generally comprised as pathogen impermeable and compatible with one or more of radiation imaging and magnetic resonance (MR) imaging systems and an air filtration system configured to supply filtered cooling air flow to an interior of the imaging system isolation enclosure and the imaging system.

In contrast to known medical equipment pathogen enclosures, the disclosed novel imaging system isolation enclosure is configured for use with all medical imaging systems. Although a CT imaging system is shown and described by way of example in FIG. 1, it should be understood that the patient isolation unit may also be used with other imaging systems, such as an X-ray imaging system, a CT imaging system , a positron emission tomography (PET) imaging system, a single-photon emission computerized tomography (SPECT) imaging system, a MR imaging system, and combinations thereof (e.g., multi-modality imaging systems, such as PET/CT, PET/MR or SPECT/CT imaging systems). The present discussion of a CT imaging system is provided merely as an example of one suitable imaging modality. The imaging system isolation enclosure provides isolation of the imaging system from the surrounding imaging room or suite, operators, technologists, nursing aids, nurses, physicians and/or other healthcare professionals.

Those trained in the art will appreciate that, within a CT imaging system's gantry enclosure assembly 102, the air flow is complex due to the turbulence introduced by rotation of the gantry. However, within any imaging system's gantry volume, whether it be CT, PET, SPECT, MR, or other, cooling air must be circulated through the gantry volume to remove heat produced by the imaging system's components. A net pressure differential is required to cause air to flow through the gantry volume; airflow is commonly produced using one or more exhaust fans. At the air intake, the pressure must be negative with respect to the surrounding ambient (e.g., the imaging room or suite); furthermore, to maintain net air flow toward the exhaust, the pressure within the gantry volume but near the exhaust must be negative with respect to the pressure near the intake. Therefore, the average pressure within the gantry must be negative with respect to the surrounding ambient and, if the gantry is not airtight, pathogens can enter the gantry enclosure assembly at air leaks. However, with an airtight gantry enclosure assembly, no pathogens can enter the internal gantry volume except at a provided air intake.

An embodiment of a imaging system isolation enclosure 110 as illustrated in FIG. 1, includes a pathogen impermeable gantry enclosure assembly 102 generally comprised of an substantially airtight gantry enclosure assembly provided at the time of manufacture or as a field upgrade to the imaging system, such that airtight seals are provided between a plurality of sections of the gantry enclosure assembly. In an alternate embodiment, the pathogen impermeable enclosure may be formed as an airtight singular component at the time of manufacture. The imaging system isolation enclosure 110 includes an airtight seal between the gantry enclosure assembly and the support surface of the imaging system, such as a floor of the imaging suite (described presently). The imaging system isolation enclosure 110, and more particularly, the gantry enclosure assembly or enclosure includes an air handling system 120 including at least one inlet 116 to supply a cooling air flow to an interior of the imaging system isolation enclosure 110 and at least one outlet 112, 114 to output exhaust air from an interior of the imaging system isolation enclosure. In an exemplary embodiment, one or more air intake filters are provided to permit cooling air to enter the imaging system isolation enclosure, and more particularly, the gantry enclosure assembly 112, in a controlled manner and to prevent pathogens and/or vaporized sterilization solutions from entering the internal volume of the imaging system isolation enclosure. During use of the imaging system, the intake cooling air flow enters the imaging system isolation enclosure via the one or more filters at the air intake. The intake cooling air flow passes into an interior of the imaging system isolation enclosure, and more particularly, the gantry enclosure assembly. One or more imaging system fans may provide for assistance in exhausting the intake air flow.

Airtight seals may be constructed of any suitable material such as rubber, plastic, or closed-cell foam and provided at the time of manufacture or may be provided as a field upgrade to the imaging system. In an exemplary embodiment, protective covers for controls and/or displays may be provided. Such protective covers may be transparent and/or flexible to permit visualizing and/or physically interacting with said controls and/or displays. Such protective covers may be fastened with any suitable means such as adhesive, tape, hook-and-loop fasteners, rubber zippers, and the like.

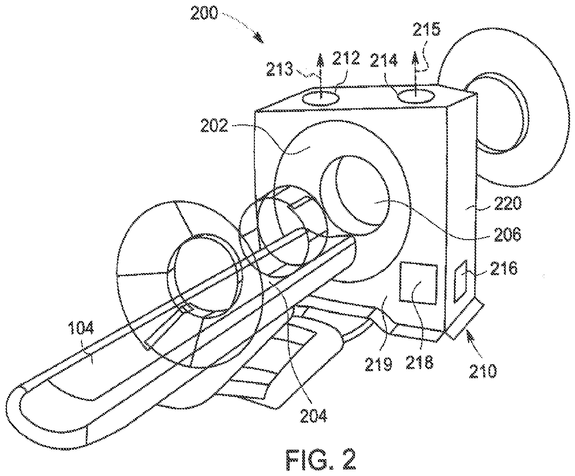

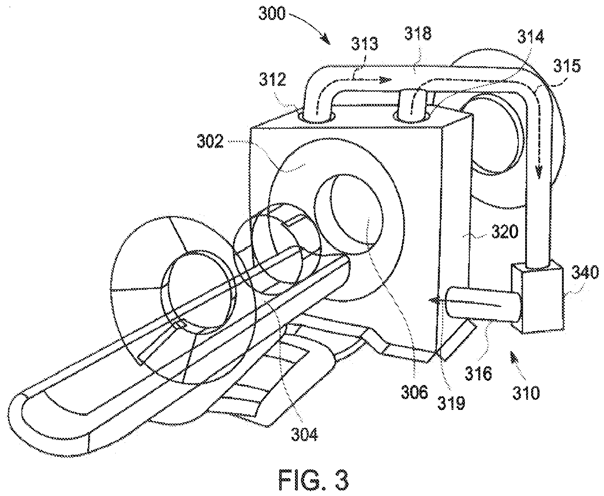

A imaging system isolation enclosure embodiment, as illustrated in FIGS. 2 and 3, includes a pathogen impermeable enclosure generally comprised of a jacket, a plurality of conical components coupled thereto, a cylindrical bore tube coupled to the plurality of conical components and an air handling system including an inlet to supply a cooling air flow to an interior of the imaging system isolation enclosure and an outlet to output exhaust air from an interior of the imaging system isolation enclosure. The imaging system isolation enclosure is generally sized to provide for placement in a manner to enclose a gantry of the medical imaging system. In an exemplary embodiment, the jacket may include openings to which the plurality of conical components are coupled. Coupling may include any means that provides an airtight seal between the jacket and each of the plurality of conical components, including, but not limited to, chemical welding, heat welding, tape, or the like. In addition, the plurality of conical components are sealingly coupled to the cylindrical bore tube, such as by taping. The assembled imaging system isolation enclosure may further be sealingly coupled to a support surface, about a perimeter of the imaging system isolation enclosure, such as the imaging suite floor, using any coupling means that provides an airtight seal. In an exemplary embodiment, the imaging system isolation enclosure may be sealed to the floor using tape, hook-and-loop fasteners (Velcro^{®}), rubber zippers with one half fused to the imaging system isolation enclosure and the other half attached to the floor using tape or adhesive, and the like. In an exemplary embodiment, the imaging system isolation enclosure may extend to encompass an imagining table base, or the complete imaging table. Portions of the imaging table base, and/or cables present between the imaging table base and the gantry enclosure assembly may be sealed at an interface with the imaging system isolation enclosure. In an exemplary embodiment, to minimize, if not eliminate, any potential leak at the table to gantry or cable to gantry interface, the cover over the connection between the gantry and the patient table may be removed and filled with any suitable material prior to repositioning said cover; suitable means to seal the gantry to table interface may include but are not limited to one or more inflatable bladders, clay, duct seal, or other suitable flexible solid material, or a fine particulate media that is selected to act as a filter and be of a material that is unfriendly to pathogens.

In an exemplary embodiment, the imaging system isolation enclosure may be formed as a completely rigid gantry enclosure assembly, a completely flexible gantry enclosure assembly, or a combination with portions of the cover assembly being rigid and other portions being flexible. For example, in an exemplary embodiment, a front face of the gantry may be covered with a flexible material, and all other faces covered with a single rigid cover assembly component that is pushed onto the gantry from the back side and coupled thereto the front covering to form a imaging system isolation enclosure.

In an exemplary embodiment, the jacket portion of the imaging system isolation enclosure is generally shaped as a five-sided box like structure and comprised of a material suitable for placement about the medical imaging system, such as, but not limited to any suitable pathogen impermeable material, such as a biochemically resistive material. In an exemplary embodiment, the jacket may exclude use of magnetic material, such as would be required for MR imaging systems. In an exemplary embodiment, the jacket may be formed of polyethylene, polyurethane, polyvinyl chloride (vinyl), or the like. In one particular embodiment, the jacket is formed of a vinyl material, such as a 10 to 50 mil thick vinyl material. In an exemplary embodiment, at least a portion of the jacket may be transparent, to enable visual positioning and/or contact with the gantry and any included control or display components.

In an exemplary embodiment, each of the plurality of conical components of the is generally shaped and sized for positioning relative to the conical front and rear portions of the gantry and the bore formed therethrough. Each of the plurality of conical components is comprised of a material suitable for placement about the medical imaging system, such as, but not limited to any suitable pathogen impermeable material, such as a biochemically resistive material. In an exemplary embodiment, each of the plurality of conical components may exclude use of magnetic material, such as would be required for MR imaging systems. In an exemplary embodiment, each of the plurality of conical components may be formed of polyethylene, polyurethane, polyvinyl chloride (vinyl), or the like. In one particular embodiment, the plurality of conical components are formed of a vinyl material, such as a 10 to 50 mil thick vinyl material. As previously indicated, each of the plurality of conical components and the jacket are sealingly coupled to one another.

In an exemplary embodiment, the cylindrical bore tube of the imaging system isolation enclosure is shaped and sized for positioning relative to the scanning window within the bore of the gantry. The cylindrical bore tube is comprised of a non-metallic material suitable for placement within the bore of the medical imaging system, such as, but not limited to any suitable pathogen impermeable transparent material, such as a biochemically resistive material. The cylindrical bore tube is formed of a material that is transparent and of substantially uniform thickness to permit passage of visible light without optical distortion passing therethrough the scanning window during the imaging procedure. In an exemplary embodiment, the cylindrical bore tube of the imaging system isolation enclosure is formed of a transparent material, such as a Mylar^{®}.

In an alternate embodiment, the components of the imaging system isolation enclosure may be coupled one to another, to the imaging system, and/or to the support surface utilizing strips of hook and loop fasteners, rubber zippers, or any known fastener, suitable to provide a substantially airtight seal, and compatible for use in an imaging system. In an exemplary embodiment, the fasteners may exclude use of magnetic material, such as would be required for MR imaging systems.

The imaging system isolation enclosure further includes an air handling system that provides for the intake and exhaust of a cooling air flow passing therethrough the gantry. In an exemplary embodiment, the jacket may include one or more intake filters, such as one or more high efficiency particulate air (HEPA), electrostatic filters, or the like, capable of filtering pathogens present in the air intake. The intake filters may be integrally formed with the jacket. In an alternate embodiment, the jacket may include a filter frame that is sealingly coupled to the jacket and into which replaceable filters may be positioned. The air handling system further includes openings to provide for exhaust of the cooling air flow from within the imaging system. In an exemplary embodiment, the exhaust openings may include one or more filters or fans as required.

During use of the imaging system, the intake cooling air flow enters the imaging system isolation enclosure via the one or more filters at the air intake. The intake cooling air flow passes in a space defined between the imaging system isolation enclosure and the gantry enclosure assembly and into an interior of the gantry enclosure assembly via a space defined, such as between the gantry enclosure assembly and the support surface, such as the floor. The imaging system isolation enclosure air handling system provides for normal image system function with the cooling flow being unimpaired. One or more supplemental fans may provide for assistance in exhausting the cooling air flow.

In an exemplary embodiment, the imaging system isolation enclosure's intake air filter technology is selected to inhibit passage of vaporized H₂O₂ solution or similar sterilization technology. In this embodiment, subsequent to imaging system use, the imaging system isolation enclosure allows for the imaging equipment and imaging suite to be disinfected as dictated by specific pathogen contamination, such as by using H₂O₂ fogging or similar technology, without concern about damage to the imaging equipment's internal components, while the imaging equipment is powered on and the cooling air exhaust fans are running. Suitable intake air filter technologies may include those that permit continued use of the filters immediately after the sterilization procedure, technologies that require replacing the filters after the sterilization procedure, or those that permit continued or reuse of the filters after condensed sterilization solution has evaporated.

A imaging system isolation enclosure embodiment, as illustrated in FIG. 3, includes a pathogen impermeable enclosure generally similar to the previous embodiment, but in contrast, includes a closed loop air handling system. More particularly, to provide cooling to the gantry components, an air conditioning unit is provided and coupled to an air intake and exhaust outflow of the jacket. In an exemplary embodiment, during operation, the air conditioning unit provides air to air heat transfer from the air flow exhausted by the imaging equipment to the surrounding environmental air. In an exemplary embodiment, the air conditioning unit is sealingly coupled to the jacket via an elongated conduit, such as pathogen impermeable flexible hose, that may include reinforcements if required. In an exemplary embodiment, the conduit may be coupled to the jacket via chemical welding, heat welding, use of rubber zipper, or the like, to facilitate a pathogen impermeable seal therebetween. In an exemplary embodiment, the air to air heat transfer may be achieved by means of an independent, electrically powered air conditioner. In an alternative embodiment, the air to air heat transfer may be achieved by means of a heat exchanger that transfers heat to a liquid cooling loop, such as may be provided by a chilled water or other liquid cooling source that may be provided or that may be available in the healthcare facility. In an exemplary embodiment, the air handling system may further include a collection tank for the removal of condensate, such as a dehumidifier, and/or additional fans to assist with the flow of air through the imaging system isolation enclosure and the imaging equipment. In this particular embodiment, the imaging system isolation enclosure including a closed loop air handling system minimizes, if not eliminates, exposure of the gantry to pathogens present in the imaging suite, and also minimizes, if not eliminates, exposure of the gantry to sterilization processes such as H₂O₂ fogging or similar technology.

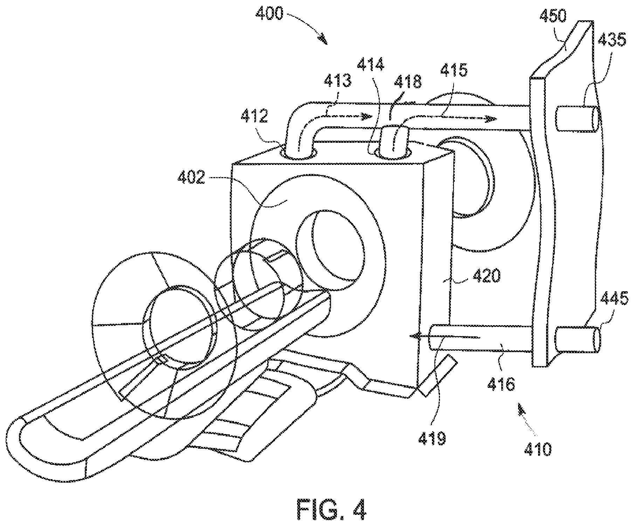

A imaging system isolation enclosure embodiment, as illustrated in FIG. 4, includes a pathogen impermeable enclosure generally similar to the embodiment of FIGS. 1 and 2, but in contrast, includes an air handling system that provides for the intake of clean air from an uncontaminated air flow source, such as another room or exterior space, and the exhaust of the cooling air flow to a separate room, or exterior space. More particularly, to provide cooling to the gantry components, a conduit in fluid communication with a clean air source is coupled to an air intake on the jacket of the imaging system isolation enclosure. A separate conduit in fluid communication with a separate room or exterior space may be coupled to an output of the exhaust outflow on the jacket of the imaging system isolation enclosure. During operation a cooling air flow is input to the imaging equipment and exhausted by the imaging equipment via the conduits. In an exemplary embodiment, the conduits are sealingly coupled to the jacket and formed of a pathogen impermeable material, such as a flexible hose, that may include reinforcements if required. In an exemplary embodiment, the conduits may be coupled to the jacket via chemical welding, heat welding, use of rubber zipper, or the like, to facilitate a pathogen impermeable seal therebetween. In an exemplary embodiment, the air handling system may further include additional fans to assist with the flow of air through the air handling system. In this particular embodiment, the imaging system isolation enclosure air handling system minimizes, if not eliminates, exposure of the gantry to pathogens present in the imaging suite, and also minimizes, if not eliminates, exposure of the gantry to sterilization processes such as H₂O₂ fogging or similar technology.

For any of the preceding embodiments, user interface with the imaging system may be remote controlled. As an example, pushbutton controls, lighted indicators, and/or display screens may be replaced or supplemented with wired or wireless controls that extend imaging system control functions that were provided on the imaging system's gantry to other locations, such as the imaging system's control room.

Accordingly disclosed is a imaging system isolation enclosure that provides isolation from pathogens in a surrounding environment, during medical imaging procedures. The imaging system isolation enclosure may be configured to provide a barrier and isolate the imaging system from a patient with an infectious disease and/or isolate the imaging system from pathogens in the imaging suite. The novel imaging system isolation enclosure is comprised of materials compatible with radiation imaging (e.g., no metal or other dense objects in the X-ray beam) and MRI (e.g., no magnetic objects). The imaging system isolation enclosure includes a filtered, closed-loop or clean air intake air handling system that provides for the intake of clean air within the imaging system isolation enclosure to ensure no pathogen enter the imaging system isolation enclosure to contaminate the imaging system, while not inhibiting the system's required cooling air flow volume. The disclosed novel imaging system isolation enclosure eliminates imaging equipment contamination problems, while enabling fogging to decontaminate the imaging suite without concern for the imaging equipment. The imaging system isolation enclosure as disclosed provides economic and health benefits in a reduction in the workload associated with scanning infectious patients, increases throughput, and reduces exposure to hospital personnel and non-infectious patients. The imaging system isolation enclosure may be sized and shaped for compatibility with a gantry bore of most, if not all, medical imaging system. The imaging system isolation enclosure as disclosed prevents contamination of the imaging equipment, prevents recirculation of pathogens in the imaging suite and enables servicing of the imaging equipment without concern for pathogens that may have penetrated the gantry.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

While the present disclosure has been described with reference to one or more drawings and exemplary embodiments, it will be understood by those skilled in the art that various changes may be made and equivalents may be substituted for elements thereof without departing from the scope of the disclosure. In addition, many modifications may be made to adapt a particular situation or material to the teachings of the disclosure without departing from the essential scope thereof. Furthermore, the skilled artisan will recognize the interchangeability of various features from different embodiments. For example, various features described, as well as other known equivalents for each feature, may be mixed and matched by one of ordinary skill in this art to construct additional systems and techniques in accordance with principles of this disclosure. Therefore, it is intended that the disclosure not be limited to the particular embodiments disclosed as the best mode. This written description uses examples to disclose the invention, including the best mode, and also to enable any person skilled in the art to practice the invention, including making and using any devices or systems and performing any incorporated methods.

Embodiments of the present disclosure shown in the drawings and described above are example embodiments only and are not intended to limit the scope of the appended claims, including any equivalents as included within the scope of the claims. Various modifications are possible and will be readily apparent to the skilled person in the art. It is intended that any combination of non-mutually exclusive features described herein are within the scope of the present invention. That is, features of the described embodiments can be combined with any appropriate aspect described above and optional features of any one aspect can be combined with any other appropriate aspect. Similarly, features set forth in dependent claims can be combined with non-mutually exclusive features of other dependent claims, particularly where the dependent claims depend on the same independent claim. Single claim dependencies may have been used as practice in some jurisdictions require them, but this should not be taken to mean that the features in the dependent claims are mutually exclusive.

## Claims

1. An imaging system isolation enclosure (110) comprising:
a pathogen impermeable enclosure for use with one or more of radiation imaging systems and magnetic resonance imaging systems, the pathogen impermeable enclosure configured to provide a barrier between the imaging system and at least one of a patient user and an imaging room;
**characterised in that**
an air filtration system including an inlet (116) to supply a cooling air flow to an interior of the imaging system isolation enclosure and an outlet (112, 114) to output exhaust air from an interior of the imaging system isolation enclosure, the air filtration system preventing pathogens and/or vaporized sterilization solutions from entering the internal volume of the imaging system isolation enclosure.

2. The imaging system isolation enclosure of claim 1, wherein the pathogen impermeable enclosure comprises:
a flexible jacket;
a plurality of conical components sealingly coupled to the jacket; and
a cylindrical bore tube sealingly coupled to the plurality of conical components.

3. The imaging system isolation enclosure of claim 1, wherein the pathogen impermeable enclosure comprises a gantry enclosure assembly (102).

## Patentansprüche

1. Bildgebungssystemisolationsgehäuse (110), welches umfasst:
ein für Krankheitserreger undurchlässiges Gehäuse zur Verwendung mit einem oder mehreren Strahlungsbildgebungssystemen und Magnetresonanzbildgebungssystemen, wobei das für Krankheitserreger undurchlässige Gehäuse dafür ausgelegt ist, eine Barriere zwischen dem Bildgebungssystem und einem Patienten und/oder einem Bildgebungsraum bereitzustellen;
**gekennzeichnet durch**
ein Luftfiltrationssystem, das einen Einlass (116) aufweist, um einen Kühlluftstrom einem Inneren des Bildgebungssystemisolationsgehäuses zuzuführen, und einen Auslass (112, 114), um Abluft aus einem Inneren des Bildgebungssystemisolationsgehäuses abzuführen, wobei das Luftfiltrationssystem verhindert, dass Krankheitserreger und/oder verdampfte Sterilisationslösungen in das Innenvolumen des Bildgebungssystemisolationsgehäuses eintreten.

2. Bildgebungssystemisolationsgehäuse nach Anspruch 1, wobei das für Krankheitserreger undurchlässige Gehäuse umfasst:
einen flexiblen Mantel;
mehrere konische Komponenten, die mit dem Mantel dichtend gekoppelt sind; und
ein Rohr mit zylindrischer Bohrung, das mit den mehreren konischen Komponenten dichtend gekoppelt ist.

3. Bildgebungssystemisolationsgehäuse nach Anspruch 1, wobei das für Krankheitserreger undurchlässige Gehäuse eine Gantrygehäuseanordnung (102) umfasst.

## Revendications

1. Enceinte d'isolation de système d'imagerie (110) comprenant :
une enceinte imperméable aux pathogènes destinée à être utilisée avec un ou plusieurs systèmes d'imagerie par rayonnement et systèmes d'imagerie par résonance magnétique, l'enceinte imperméable aux pathogènes étant configurée pour fournir une barrière entre le système d'imagerie et au moins une partie parmi un utilisateur patient et une salle d'imagerie ;
**caractérisée en ce qu'**un système de filtration d'air comprenant une entrée (116) pour fournir un flux d'air de refroidissement à l'intérieur de l'enceinte d'isolement de système d'imagerie et une sortie (112, 114) pour évacuer l'air d'échappement de l'intérieur de l'enceinte d'isolement de système d'imagerie, le système de filtration d'air empêchant l'entrée de pathogènes et/ou de solutions de stérilisation vaporisées dans le volume interne de l'enceinte d'isolement de système d'imagerie.

2. Enceinte d'isolement de système d'imagerie selon la revendication 1, dans laquelle l'enceinte imperméable aux pathogènes comprend :
une chemise flexible ;
une pluralité de composants coniques couplés de manière étanche à la chemise ; et
un tube d'alésage cylindrique couplé de manière étanche à la pluralité de composants coniques.

3. Enceinte d'isolement de système d'imagerie selon la revendication 1, dans laquelle l'enceinte imperméable aux pathogènes comprend un ensemble d'enceinte à portique (102).
